# EUROPEAN PATENT APPLICATION

(11) **EP 4 613 269 A1**
(43) Date of publication of application: **10.09.2025**
(21) Application number: 24161537.6
(22) Date of filing: 05.03.2024
(51) Int. Cl.: A61K 31/437, A61K 9/28, A61P 19/00

(54) **RIFAXIMIN FOR USE IN TREATING SARCOPENIA IN PATIENTS WITH LIVER DISEASE**

(71) Applicant: Norgine BV, 1083 HP Amsterdam (NL)
(72) Inventor: WORLAND, Thomas, Melbourne (AU); HEY, Penelope, Melbourne (AU); WONG, Darren, Melbourne (AU); APOSTOLOV, Ross, Melbourne (AU); CHAN, R. Kimberley, Melbourne (AU); SINCLAIR, Marie, Melbourne (AU); GOW, Paul, Melbourne (AU)
(74) Representative: Carpmaels & Ransford LLP

(57) **Abstract**

The present invention relates to rifaximin for use in methods of treating or preventing sarcopenia in a human patient with liver disease, such as a patient with cirrhosis.

## Description

### FIELD OF THE INVENTION

The present invention relates to rifaximin for use in methods of treating or preventing sarcopenia in a human patient with liver disease, such as a patient with cirrhosis.

### BACKGROUND TO THE INVENTION

Sarcopenia, defined as the loss of skeletal muscle mass, strength and physical function, is a common skeletal muscle abnormality in patients with liver disease, which is associated with a high incidence of complications both pre- and post-liver transplantation. Sarcopenia is an important prognostic factor in cirrhosis and is estimated to occur in up to 70% of patients with advanced liver disease. Muscle depletion is associated with a two-fold higher mortality rate in patients with cirrhosis, independent of liver disease severity. The mechanisms underlying sarcopenia in liver disease are multifactorial with major contributors being malnutrition, systemic inflammation, hormonal dysregulation (including insulin-like growth factor-1 (IGF-1)) and hyperammonemia.

While the clinical implications of sarcopenia in liver disease (and, in particular, cirrhosis) are well documented, there are limited data on treatment strategies for sarcopenia in this population. Exercise, particularly resistance training, has been considered the main strategy to prevent sarcopenia in various populations. Nutritional interventions, such as recommended daily energy and protein intakes, to support resistance exercise have also attracted significant attention in recent years for patients with chronic liver diseases. Nutritional supplements have been investigated in small clinical trials or pilot studies. Supplementation of branched chain amino acids (BCAAs) has been investigated, but data to support their efficacy in cirrhosis are currently lacking. L-carnitine has also been explored as a nutritional supplement.

Accordingly, there remains a large unmet need for safe and effective therapies to mitigate the loss of muscle mass in patients with liver disease.

### SUMMARY OF THE INVENTION

The inventors have found that rifaximin administration is associated with an increase in muscle mass (as measured by an increase in SMA) in patients with liver disease. Such an effect demonstrates that rifaximin can be used to treat or prevent sarcopenia in this patient group, for example leading to improved muscle mass, muscle strength and/or muscle function.

Thus, in one aspect the invention provides rifaximin for use in a method of treating or preventing sarcopenia in a human patient with liver disease, such as a patient with cirrhosis.

In another aspect, the invention provides a method of treating or preventing sarcopenia in a human patient with liver disease (such as a patient with cirrhosis), wherein the method comprises administering rifaximin to the patient.

In a further aspect, the invention provides a use of rifaximin in the manufacture of a medicament for treating or preventing sarcopenia in a human patient with liver disease.

In some embodiments, the human patient has cirrhosis. In some embodiments, the patient has hepatic encephalopathy. In some embodiments, the human patient has cirrhosis and hepatic encephalopathy. In some embodiments, the method comprises administering rifaximin at a dose of between about 100 mg and about 1000 mg, between about 200 mg and about 900 mg, between about 300 mg and about 800 mg, between about 400 mg and about 700 mg, and between about 500 mg to about 600 mg. In some embodiments, the method comprises administering rifaximin at a dose of about 550 mg. In some embodiments, the method comprises administering a dose of about 550 mg rifaximin once, twice or three times daily. In some embodiments, the method comprises administering a dose of about 550 mg rifaximin twice daily. In some embodiments, the rifaximin is administered in tablet form, preferably a film-coated tablet. In some embodiments, the tablet core consists of the excipients sodium starch glycolate type A, glycerol distearate, colloidal anhydrous silica, talc and microcrystalline cellulose. In some embodiments, the film coat of the film-coated tablet consists of hypromellose, titanium dioxide, disodium edetate, propylene glycol and red iron oxide. In some embodiments, the patient is an adult, optionally an adult aged 50 years or older. In some embodiments, the method results in at least maintenance of skeletal muscle area. In some embodiments, the method results in an increase in skeletal muscle area of at least about 0.25 cm², at least about 0.50 cm², or at least about 0.70 cm². In some embodiments, the method results in an increase in skeletal muscle area of at least 0.25%, at least 0.50%, at least 0.75%, at least 1.0% or at least 2.0%, preferably at least 0.50%. In some embodiments, the method further comprises administering lactulose to the patient.

In preferred embodiments, the human patient has cirrhosis and hepatic encephalopathy, the method comprises administering a dose of about 550 mg rifaximin twice daily and the method results in an increase in skeletal muscle area of at least about 0.25 cm², at least about 0.50 cm², or at least about 0.70 cm² (wherein SMA is the skeletal muscle cross-sectional area at the level of the third lumbar (L3) vertebra derived from a CT scan and the change in SMA is determined by comparing first and second CT scans), and wherein optionally the method further comprises administering lactulose to the patient.

In preferred embodiments, the human patient has cirrhosis and hepatic encephalopathy, the method comprises administering a dose of about 550 mg rifaximin twice daily and the method results in a percentage increase in SMA of at least 0.25%, at least 0.50%, at least 0.75%, at least 1.0%, or at least 2.0%, preferably at least 0.50%. (wherein SMA is the skeletal muscle cross-sectional area at the level of the third lumbar (L3) vertebra derived from a CT scan and the change in SMA is determined by comparing first and second CT scans), and wherein optionally the method further comprises administering lactulose to the patient.

### DESCRIPTION OF FIGURES

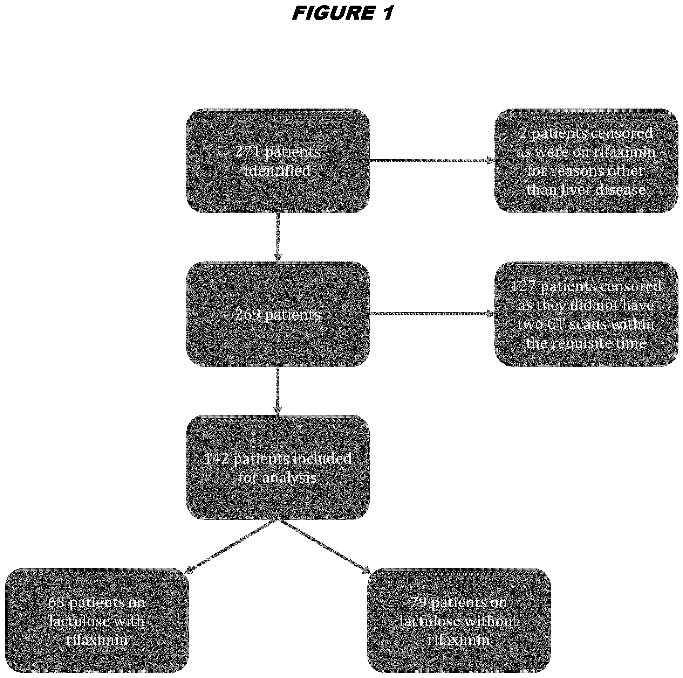

**Figure 1****.** Patient inclusion flowchart, showing the inclusion criteria and the 142 patients that met the inclusion criteria and were included in this study.

### DETAILED DESCRIPTION

The invention relates to rifaximin for use in methods of treating or preventing sarcopenia in a human patient with liver disease, such as a patient with cirrhosis.

### Rifaximin

Rifaximin is a semisynthetic broad spectrum antibacterial drug, derived through chemical modification of the natural antibiotic rifamycin. Chemical names for rifaximin include 4-deoxy-4'-methylpyrido[1',2'-1,2]imidazo-[5,4-c] rifamycin SV and
(2S,16Z,18E,20S,21S,22R,23R,24R,25S,26S,27S,28E)-5,6,21,23,25-pentahydroxy-27-methoxy-2,4,11,16,20,22,24,26-octamethyl-2,7-(epoxypentadeca-[1,11,13]trienimino)benzofuro[4,5-e]pyrido[1,2-a]-benzimidazole-1,15(2H)-dione,25-acetate. Its chemical structure is provided below.

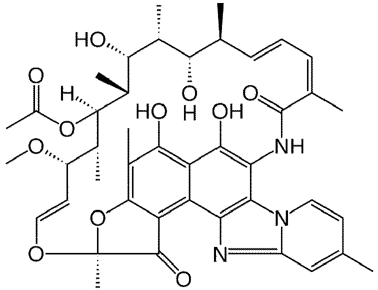

Rifaximin has broad spectrum antibacterial properties against both Gram-positive and Gram-negative anaerobic and aerobic bacteria. It acts as an antibiotic by inhibiting RNA synthesis in susceptible bacteria by binding to the beta subunit of bacterial RNA polymerase enzyme. This binding blocks translocation, which stops transcription. Rifaximin has very low bioavailability due to its poor absorption after oral administration. Most of the drug taken orally stays in the gastrointestinal tract. As a result of bile acid solubility, its antibacterial action is limited mostly to the small intestine and less so the colon.

Rifaximin is authorized for the treatment of travelers' diarrhea, irritable bowel syndrome and hepatic encephalopathy.

The reference to "rifaximin" herein is shorthand for "rifaximin or a pharmaceutically acceptable salt and/or solvate thereof'. The term "solvate" is used herein to describe a molecular complex comprising rifaximin and a one or more pharmaceutically acceptable solvent molecules, for example, ethanol or water. The term "hydrate" is employed when the solvent is water and for the avoidance of any doubt, the term "hydrate" is encompassed by the term "solvate". Several distinct crystalline forms of rifaximin have been reported (e.g. alpha, beta, gamma, delta, epsilon, kappa, tau etc.). These are essentially non-stoichiometric hydrates that interconvert depending on the amount of water and on the way dehydration/hydration is carried out. The various forms of rifaximin can exhibit differences in water solubility (e.g. from 2 mg/L up to more than 40 mg/L), which can impact rifaximin bioavailability. Identification of the various rifaximin hydrates can be carried out by powder X-ray diffraction. Thus, the reference to "rifaximin" herein includes various rifaximin hydrate forms.

In some embodiments, the rifaximin is in the alpha polymorphic form (or consists essentially of rifaximin in the alpha polymorphic form).

Herein "pharmaceutically acceptable salt" means a physiologically or toxicologically tolerable salt and includes pharmaceutically acceptable acid addition salts. For example where a compound contains a basic group, such as an amino group, pharmaceutically acceptable acid addition salts that can be formed include hydrochlorides, hydrobromides, sulfates, phosphates, acetates, citrates, lactates, tartrates, mesylates, succinates, oxalates, phosphates, esylates, tosylates, benzenesulfonates, naphthalenedisulphonates, maleates, adipates, fumarates, hippurates, camphorates, xinafoates, p-acetamidobenzoates, dihydroxybenzoates, hydroxynaphthoates, succinates, ascorbates, oleates, bisulfates and the like. For a review of suitable salts, see "Handbook of Pharmaceutical Salts: Properties, Selection and Use" by Stahl and Wermuth (Wiley-VCH, Weinheim, Germany, 2002).

### Treating or preventing sarcopenia in liver disease

The invention relates to treating or preventing sarcopenia in human patients with liver disease. Herein it is shown that rifaximin treatment is associated with improved muscle mass (SMA) in such patients.

Sarcopenia is a term derived from the Greek sarco (flesh) and penia (deficiency). Sarcopenia was initially defined as age-related loss of skeletal muscle, but has since been expanded to reflect low skeletal muscle mass leading to negative effects on physical performance and clinical outcomes across a broad range of disease states outside of geriatric populations.

Measurements of skeletal muscle cross-sectional area (SMA) at the level of the third lumbar (L3) vertebra derived from computed tomography (CT) scans are commonly used in assessments of sarcopenia, as is skeletal muscle index (SMI), which is the height-normalised SMA.

Herein, a patient with sarcopenia has a skeletal muscle index (SMI) of <50 cm²/m² in men and <39 cm²/m² in women.

Patients with sarcopenia may also have reduced muscle strength and/or muscle function. Suitable tests and their cut offs for determining low muscle strength and low muscle function are provided in the table below.

| **Criteria** | **Test and cut off** |
|---|---|
| Low muscle strength | Grip strength (males) < 27 kg |
| | Grip strength (females) < 16 kg |
| | Chair standing > 15 s for five rises |
| Low muscle function | Gait speed ≤ 0.8 m/s |
| | Short Physical Performance Battery (SPPB) ≤ 8 points score |
| | Timed Up-and-Go Test ≥ 20 s |
| | 400 m walk test, noncompletion or ≥6 min for completion |

The terms "treatment," "treat," and "treating" relates to any action that alleviates sarcopenia (e.g. by reversing a loss of skeletal muscle mass, muscle strength and/or muscle function) or inhibits worsening of sarcopenia (e.g. by maintaining the patient's muscle mass, muscle strength and/or muscle function). Treatment may also be continued after symptoms have resolved, for example to prevent or delay their recurrence.

The terms "prevention", "prevent", "preventing" or "preventive" relate to any action that suppresses or delays the onset of muscle loss, muscle strength and/or muscle function and thus the development of sarcopenia in a patient at risk thereof.

Thus, in some embodiments, the method results in maintenance of, or improvement in, skeletal muscle area. An increase in (or maintenance of) skeletal muscle can be measured using the skeletal muscle cross-sectional area (SMA) at the level of the third lumbar (L3) vertebra derived from a computed tomography (CT) scan. A change in SMA can be assessed by comparing the SMA determined from the first and second abdominal CT scans. Using this method, the inventors have shown an increase in SMA associated with rifaximin administration (a median change in SMA of +0.74 cm², which corresponds with an increase in muscle mass of about 0.5%) . Thus, in some embodiments of the invention, the method results in an increase in SMA of at least about 0.25 cm², at least about 0.50 cm², or at least about 0.70 cm² (wherein SMA is the skeletal muscle cross-sectional area at the level of the third lumbar (L3) vertebra derived from a CT scan and the change in SMA is determined by comparing first and second CT scans).

In some embodiments, the method results in maintenance of SMA in a patient with sarcopenia (i.e. substantially no change between first and second CT scans). In some embodiments, the method results in maintenance of SMA in a patient at risk of development of sarcopenia (i.e. substantially no change between first and second CT scans). Maintenance of muscle mass is beneficial because, for example, progressive worsening of sarcopenia in patients is prevented.

An increase in muscle mass may also be defined as a percentage increase in SMA of the patient between first and second abdominal CT scans using the skeletal muscle cross-sectional area at the level of the third lumbar (L3) vertebra. A percentage change of more than 0.25% may represent an improvement in muscle mass in a patient with sarcopenia. In some embodiments of the invention, the method results in a percentage increase in SMA of at least 0.25%, at least 0.50%, at least 0.75%, at least 1.0%, at least 1.5%, or at least 2.0%. In preferred embodiments, the method results in a percentage increase in SMA of at least 0.5%.

When measuring an increase in, or maintenance of, SMA according to the methods above, the first and second CT scans may be separated by a period of about 1 month to about 15 months or a period of about 2 months to about 12 months. In some embodiments, the first and second CT scans are separated by a period of about of about 6 months to about 8 months. The median time period between the first and second CT scans of the patients in the examples was 216 days (around 7 months).

A change in muscle strength and/or muscle performance may be measured by comparing the outcome of first and second assessments carried out at separate time points according to the table above. An improvement in muscle strength or muscle function may be identified if the patient is found to have low muscle strength and/or low muscle function at the first assessment, and at the second assessment the patient no longer fulfils one or more (and preferably all) of the tests for low muscle strength and/or low muscle function.

### Liver disease

"Liver disease" or hepatic disease, is any of many diseases of the liver. Liver disease can be acute or chronic. Acute liver failure is loss of liver function that occurs quickly (days or weeks), usually in a person who has no preexisting liver disease. It is most commonly caused by a hepatitis virus or drugs, such as acetaminophen.

Chronic liver disease (CLD) is a progressive deterioration of liver functions for more than six months. The most common etiologies are alcoholic liver disease, non-alcoholic fatty liver disease (NAFLD or NASH), chronic viral hepatitis, genetic causes and autoimmune causes. Alcoholic liver disease is a spectrum of disease which includes alcoholic fatty liver with or without hepatitis, alcohol hepatitis (reversible because of acute ingestion) to cirrhosis (irreversible). Patients with severe alcohol use disorder mostly develop chronic liver disease; this is the most frequent cause of CLD. NAFLD has an association with metabolic syndrome (obesity, hyperlipidemia, and diabetes mellitus). Some of these patients develop non-alcoholic steatohepatitis, which leads to fibrosis of the liver. All the risk factors of metabolic syndrome can aggravate the disease process. Chronic viral hepatitis as a result of chronic hepatitis B, C or D infection is one of the most common causes of chronic liver disease in East Asia and Sub-Saharan Africa. Chronic hepatitis C, if not treated, may lead to hepatocellular carcinoma. Genetic causes of CLD include alpha-1 antitrypsin deficiency and hereditary hemochromatosis and Wilson disease. Alpha-1 antitrypsin deficiency is the most common genetic cause of CLD among children. Hereditary hemochromatosis is an autosomal recessive disorder of iron absorption, where a mutation involving the HFE gene that regulates the iron absorption from the intestine results in excessive iron absorption from the gastrointestinal tract. This leads to a pathological increase in total body iron, resulting the generation of hydroxyl free radicals, which in turn causes organ fibrosis. Wilson disease is an autosomal recessive disorder leading to copper accumulation. Autoimmune causes of CLD include autoimmune hepatitis (AIH), primary biliary cirrhosis (PBC) and primary sclerosing cholangitis (PSC). AIH is a rare disease and is a form of chronic inflammatory hepatitis in which the liver parenchyma is destroyed by autoantibodies. Most of the patients (more commonly females) who present with this disease have already developed cirrhosis. PBC is an autoimmune disease that involves the destruction of intrahepatic biliary channels and portal inflammation and scarring, which leads to cholestatic jaundice and fibrosis of liver parenchyma. Other causes of CLD include drugs (e.g. amiodarone, isoniazid, methotrexate, phenytoin or nitrofurantoin), vascular causes (e.g. Budd-Chiari syndrome) and idiopathic/cryptogenic causes.

In some embodiments, the patient with liver disease has chronic liver disease. For example, the patient with liver disease herein may have alcoholic liver disease, non-alcoholic fatty liver disease, or viral hepatitis.

The model for end stage liver disease (MELD) score refers to a prognostic scoring system used in assessing the severity of chronic liver disease. The MELD score estimates a patient's chances of surviving their disease during the next three months, and ranges from six (less sick) to 40 (gravely sick). As used herein, a MELD score is calculated using the following formula: (0.957 * ln(Serum Creatinine mg/dL) + 0.378 * ln(Serum Bilirubin mg/dL) + 1.120 * ln(INR) + 0.643) * 10. MELD-Na score will be calculated using the following formula: MELD - Na - [0.025 x MELD x (140- Na)] + 140, where Na is serum sodium in mmol/L.

In some embodiments, the patient has a MELD score of 10 or more, 15 or more, or 20 or more.

### Cirrhosis

In some embodiments, the patient has cirrhosis.

Cirrhosis is a final stage of chronic liver disease that results in disruption of liver architecture, the formation of widespread nodules, vascular reorganization, neo-angiogenesis, and deposition of an extracellular matrix.

For the purposes of this application, "cirrhosis of the liver" or "cirrhosis" refers to the condition in which a subject's liver is scarred and permanently damaged. Cirrhosis may be diagnosed according to methods known in the art. In one aspect, cirrhosis may be determined by one or more of histopathological evidence of cirrhosis, transient elastography, the presence of esophageal varices, and thrombocytopenia characterized by a blood platelet level of less than 150,000 in a patient with chronic liver disease. The severity of cirrhosis may also be characterized by methods known in the art. The severity of cirrhosis can be characterized by the Child-Pugh score.

The Child-Pugh scoring system (also known as the Child-Pugh-Turcotte score) determines the severity of liver disease according to the degree of encephalopathy, ascites (via clinical assessment), the serum concentrations of bilirubin and albumin, and prothrombin time (or International

Normalized Ratio; INR). Each component is given a numerical score from 1 to 3 and added together to provide total scores ranging from 5 to 15, as follows:

| **Measure** | | **1 point** | **2 points** | **3 points** |
|---|---|---|---|---|
| Total bilirubin, µmol/L (mg/dL) | | < 34 (< 2) | 34-50 (2-3) | > 50 (> 3) |
| Serum albumin, g/dL | | > 3.5 | 2.8-3.5 | < 2.8 |
| OR | Prothrombin time, prolongation (s) | < 4.0 | 4.0-6.0 | > 6.0 |
| | INR | < 1.7 | 1.7-2.3 | > 2.3 |
| Ascites | | None | Mild (or suppressed with medication) | Moderate to severe (or refractory) |
| Hepatic encephalopathy* | | None | Grade 1-2 | Grade 3-4 |

| | | | | |
|---|---|---|---|---|
| *Encephalopathy is classified as Grade 0-4 using the Conn scoring system provided below. | | | | |

A total score of 5-6 is classified as Child-Pugh grade A (well-compensated disease/good hepatic function); 7-9 is classified as Child-Pugh grade B (significant functional compromise/moderately impaired hepatic function); and 10-15 is classified as Child-Pugh grade C (decompensated disease/advanced hepatic function). In some embodiments of the invention, the patient with cirrhosis may be classified as Child-Pugh grade A, B or C. In some embodiments, the patient with cirrhosis may be classified as Child-Pugh grade B or C.

Decompensated cirrhosis or decompensated liver cirrhosis is an advanced state of cirrhosis and generally refers to an acute deterioration in liver function in a patient with cirrhosis. Decompensated cirrhosis may be characterized by one or more of jaundice, ascites, hepatic encephalopathy, hepatorenal syndrome or variceal haemorrhage. In one aspect, early decompensated liver cirrhosis refers to subjects who are diagnosed with advanced liver cirrhosis and have medically controlled ascites (>30 days) not requiring paracentesis (prophylactic variceal banding allowed if no history of previous variceal bleeding). In some embodiments, the patient with cirrhosis may have decompensated cirrhosis or may be classified as Child-Pugh grade C.

Complications of cirrhosis refer to one or more medical problems arising from the underlying disease, cirrhosis. In one aspect, complications include varices, splenomegaly, jaundice, esophageal variceal bleeding (EVB), portal hypertension ascites, edema, hepatic encephalopathy, hepatopulmonary hypertension, hepatocellular carcinoma, hepatorenal syndrome (HRS), spontaneous bacterial peritonitis (SBP), and coagulation disorders.

### Hepatic encephalopathy (HE)

In some embodiments, the patient has hepatic encephalopathy (HE). In preferred embodiments, the patient has cirrhosis and hepatic encephalopathy.

HE is a neuropsychiatric syndrome caused by hepatic dysfunction. HE may be considered "covert" or "overt" HE (CHE or OHE, respectively) depending upon the severity of the symptoms associated therewith. The Conn score, also known as the West Haven Criteria (WHC), is a 5-point scale (0 to 4) grades the severity of HE based upon neurocognitive function (e.g., consciousness, intellectual function, and behaviour). HE is defined as an altered mental status diagnosed by an increase in the Conn score to greater than Grade 2, where: Grade 0 - Minimal hepatic encephalopathy with symptoms potentially including impaired complex and sustained attention, or no personality or behavioral abnormality detected; Grade 1 (CHE) - Symptoms include trivial lack of awareness, euphoria or anxiety, shortened attention span, impairment of addition or subtraction, and altered sleep rhythm where clinical findings include mild asterixis or tremor; Grade 2 (OHE) - Symptoms include lethargy or apathy, disorientation for time, obvious personality change, and inappropriate behavior where clinical findings include obvious asterixis, dyspraxia, and slurred speech; Grade 3 (OHE) - Symptoms include somnolence to semistupor, responsive to stimuli, confused, gross disorientation, and bizarre behavior where clinical findings include muscular rigidity, clonus, and hyperreflexia; and Grade 4 (OHE) - Symptoms include coma where clinical findings include decerebrate posturing. OHE may also be observed on the Hepatic Encephalopathy Grading Instrument (HEGI), which uses clinical findings (present for at least 1 hour) to measure a patient's disorientation and thereby the severity of an HE episode (on a scale of Grade 2 to Grade 4) - Grade 4 being the most severe and Grade 2 being the least severe.

### Patient

As used herein the terms "subject" and "patient" may be used interchangeably, as the individual in need of treatment. The patient according to the invention is human. Patients of all ages can suffer from liver disease and sarcopenia. Accordingly, the human patient can be a child (ages 0 to 18 years) or an adult (18 years old or older). In preferred embodiments, the human patient is an adult aged 50 years or older.

The nutritional status of the patient may be assessed using the Subjective Global Assessment (SGA). SGA is assessed on patient history and physical examination and is scored subjectively to characterise nutritional status as well nourished (category A), mild to moderately malnourished (category B), or severely malnourished (category C) (see e.g. Detsky et al. "What is subjective global assessment of nutritional status" Journal of Parenteral and Enteral Nutrition (1987), 11(1): 8-13). In some embodiments of the invention, the patient has a subjective global assessment of category B or C.

### Dose and dosing regimen

In any of the uses or methods described herein, rifaximin is administered in a therapeutically effective amount.

A "dose" means the measured quantity of a drug to be taken at one time by a patient. Each dose administered to the patient can be sub-divided into smaller unit dosage amounts. For example, a dose of 600 mg can be sub-divided into two unit dosage amounts (e.g. two tablets), each comprising 300 mg and given at the same time.

Rifaximin can be administered at a dose of between about 100 mg and about 1000 mg, between about 200 mg and about 900 mg, between about 300 mg and about 800 mg, between about 400 mg and about 700 mg, and between about 500 mg to about 600 mg. Preferably, the dose of rifaximin is about 550 mg.

In some embodiments, rifaximin is administered twice or three times daily. Rifaximin may be administered at a dose of between about 100 mg and about 1000 mg, between about 200 mg and about 900 mg, between about 300 mg and about 800 mg, between about 400 mg and about 700 mg, and between about 500 mg to about 600 mg twice daily. Rifaximin may administered in a dose of about 550 mg once, twice or three times daily. In preferred embodiments, rifaximin is administered in a dose of about 550 mg twice daily.

"Daily dosage amount" is the total amount of drug administered in one day. The daily dosage amount can be administered as one single dose, or sub-divided into multiple doses for administration periodically during the day. Rifaximin may be administered in a daily dosage amount of between about 200 mg and about 2000 mg, between about 400 mg and about 1800 mg, between about 600 mg and about 1600 mg, between about 800 mg and about 1400 mg, and between about 1000 mg to about 1200 mg. In preferred embodiments, the daily dosage amount of rifaximin can be about 1100 mg (e.g. rifaximin is administered in a dose of 550 mg twice daily).

Unless explicitly stated to the contrary, any reference herein to a dose or dosage amount of rifaximin in mg, or amount of rifaximin in mg in any of the administration forms, should be understood as meaning the dose, dosage amount or amount in mg of rifaximin in the alpha polymorphic form (or another polymorphic form of rifaximin having the same molecular weight as rifaximin alpha), or an amount of a salt and/or hydrate (where the salt and/or hydrate has a different molecular weight) that provides an equivalent molar amount.

### Administration forms

Rifaximin administration may be in a formulation comprising one or more pharmaceutically acceptable excipients. Formulation with suitable carriers, excipients, and other agents can provide suitable transfer, delivery, tolerance, and the like. A multitude of formulations can be found in the formulary known to all pharmaceutical chemists: Remington's Pharmaceutical Sciences, Mack Publishing Company, Easton, PA.

Rifaximin is typically orally administered. The oral dosage form can be in the form of a tablet (e.g. a film-coated or sugar-coated tablet), a capsule, a granulate or a syrup. The oral dosage form can be a tablet. The tablet (core) may comprise one or more of the following excipients: sodium starch glycolate type A, glycerol distearate, colloidal anhydrous silica, talc and microcrystalline cellulose. The tablet (core) may comprise the following excipients: sodium starch glycolate type A, glycerol distearate, colloidal anhydrous silica, talc and microcrystalline cellulose. The tablet core may consist of the excipients: sodium starch glycolate type A, glycerol distearate, colloidal anhydrous silica, talc and microcrystalline cellulose.

The dosage forms described herein (e.g. the tablets) can be film coated, wherein the film coating can comprise one or more of hypromellose, lactose monohydrate, titanium dioxide and triacetin. The film coat may comprise hypromellose, titanium dioxide (E171), disodium edetate, propylene glycol and red iron oxide (E172) (opadry oy-s-34907). In preferred embodiments, the film coat consists of hypromellose, titanium dioxide (E171), disodium edetate, propylene glycol and red iron oxide (E172) (opadry oy-s-34907).

The tablet may consist of a core of rifaximin (active) and excipients sodium starch glycolate type A, glycerol distearate, colloidal anhydrous silica, talc and microcrystalline cellulose, with a film coat consisting of hypromellose, titanium dioxide (E171), disodium edetate, propylene glycol and red iron oxide (E172) (opadry oy-s-34907).

### Combination therapy

In some embodiments, rifaximin may be administered in combination with one or more further therapeutic agent(s).

The terms "combination", "in combination", "used in combination with" or "combined preparation" as used herein may refer to the combined administration of two or more agents simultaneously, sequentially or separately.

The term "simultaneous" as used herein means that the agents are administered concurrently, i.e. at the same time.

The term "sequential" as used herein means that the agents are administered one after the other.

The term "separate" as used herein means that the agents are administered independently of each other but within a time interval that allows the agents to show a combined effect in the patient.

Lactulose is a treatment for HE. In some embodiments, rifaximin may be administered in combination with lactulose. In such embodiments, rifaximin and lactulose can be administered simultaneously, sequentially or separately. Lactulose is administered in a therapeutically effective amount.

The term "therapeutically effective amount," as used herein, generally refers to an amount of a drug (e.g. rifaximin, lactulose or lactitol) sufficient to treat, ameliorate, or prevent the identified disease or condition, or to exhibit a detectable therapeutic, prophylactic, or inhibitory effect. The effect can be detected by, for example, an improvement in clinical condition, or reduction in symptoms. The precise effective amount for a subject will depend upon the subject's body weight, size, and health; the nature and extent of the condition; and the therapeutic or combination of therapeutics selected for administration. Where a drug has been approved by a regulatory authority (such as the EMA or FDA), a "therapeutically effective amount" refers to the dosage approved for treatment of the particular disease or condition.

Lactulose can be administered orally (e.g. as a syrup) or rectally (e.g. as an enema). Typically, for patients with HE it is given orally in syrup form at a dose of 15 to 30 mL two to four times a day (to aim for two semisoft stools per day).

Lactulose may be administered in an oral bolus of around 45 ml (around 30 g) and repeated hourly until the first bowel movement. This form of administration can be used for acute hepatic encephalopathy. Once the episode of encephalopathy has subsided, the dose can be titrated to achieve 2-3 soft bowel movements on a daily basis.

Lactulose may also be administered rectally at a dose of about 0.3 L in 0.7-1L of water.

In some embodiments of the uses and methods of the invention, rifaximin is administered in combination with lactitol. Lactitol is equally efficacious as, but generally better tolerated than, lactulose. Lactitol can be administered at a dose of 10 to 90 g per day to cause two soft bowel movements per day.

### Other definitions

Unless defined otherwise, all technical and scientific terms used herein have the same meaning as commonly understood to one of ordinary skill in the art to which this invention belongs.

The articles "a" and "an"" refer to one or to more than one (i.e. to at least one) of the grammatical object of the article. By way of example, "an element" means one element or more than one element.

The terms "comprise" and "comprising" are used in the inclusive, open sense, meaning that additional elements may be included.

As used herein, when a numerical value is modified by the term "about", the exact numerical value is also deemed to be disclosed.

The term "including" is used herein to mean "including but not limited to".

The term "about" in relation to a numerical value x is optional and means, for example, *x* ±10%. As used herein, when a numerical value is modified by the term "about", the exact numerical value is also deemed to be disclosed.

All publications mentioned herein are incorporated by reference in their entirety.

Embodiments provided herein may be more fully understood by reference to the following example, which is meant to be illustrative of the invention disclosed herein, but is not in any way limiting.

While examples of certain particular embodiments are provided herein, it will be apparent to those skilled in the art that various changes and modifications may be made. Such modifications are also intended to fall within the scope of the appended claims.

### EXAMPLES

This study is a single-centre retrospective cohort study of patients treated with lactulose or rifaximin with lactulose, under the care of the liver transplant service in Melbourne, Australia, between 2009 and 2022. The primary outcome measure was a change in muscle mass over time between the rifaximin versus non-rifaximin treated cohort. Secondary outcomes included. *Clostridium difficile* infection and mortality.

### Methods

### Inclusion and exclusion criteria

All adult patients (age >18 years) with cirrhosis and documented clinically apparent hepatic encephalopathy (HE) known to the Victorian liver transplant service were considered for analysis. Inclusion criteria required patients to have had serial abdominal CT scans at least 3 months and at most 9 months apart during their time on the liver transplant waitlist to allow for comparison of muscle mass over time. Rifaximin became widely available as a treatment of HE on the Pharmaceutical Benefits Scheme in Australia in January 2014. Therefore, patients on lactulose monotherapy (January 2009 to December 2013) were compared to those receiving rifaximin with lactulose (January 2014 to July 2022). Patients were orally administered 550 mg of rifaximin (alpha) twice daily. Lactulose treatment for HE was under standard conditions, i.e. lactulose administration in syrup form at a dose of 15 to 30 mL two to four times a day to aim for two semisoft stools per day. Patients otherwise received standard of care management of cirrhosis including nutritional advice from an experienced liver dietitian.

### Data collection

Initial patient identification was achieved through interrogation of the liver transplant database, a prospectively maintained database comprising all patients referred to the liver transplant unit. All patients prescribed lactulose or rifaximin with lactulose during the study period were included. All cases were reviewed and censored. Any further required data was subsequently collected from electronic medical records, electronic pathology systems, and radiology reporting software.

Included data comprised baseline demographics (age, gender, aetiology of liver disease, body mass index (BMI)); disease assessment at workup (Sodium Model of End-Stage Liver Disease (NaMELD)), Child-Pugh-Turcotte (CTP) score; sarcopenia assessment (skeletal muscle area (SMA, cm²)) from single slice CT scan, sepsis (hospitalisation data for infective complications, episodes of spontaneous bacterial peritonitis); rifaximin complications (*Clostridium difficile* infections); and mortality and transplantation outcome data.

### Primary outcome - skeletal muscle area (SMA) via abdominal CT scans

Muscle mass data was calculated from CT scans performed at two time-points in the patient journey to determine progress and change of outcomes. The first time-point for data collection, including for demographics and blood parameters, was time of first CT. The second time-point for data collection was collected between three to nine months following the first time point when the follow up CT was performed.

In patients in whom more than two CT scans were performed, the two scans that had an intervening time closest to exactly six months were chosen. SMA was measured using the cross-sectional area of the abdominal muscles at the third lumbar vertebrae (L3) using CoreSlicer (^{©}2018), a web-based program for accurate measurement of body composition, verified for this purpose (Mullie & Afilalo "CoreSlicer: a web toolkit for analytic morphomics" BMC Med. Imaging (2019), 19(1): 15).

### Clinical outcomes

Patients were followed up until: end outcomes occurred; delisting from service; rejected prior to formal transplant workup; death prior to transplant; or liver transplantation.

### Statistical analysis

Statistical analysis was performed using *R* version 4.1.1. Subgroups were compared by χ² test for categorical variables and Student's t-test or Wilcoxon rank sum test for parametric and non-parametric continuous variables, respectively. Linear regression models (analysis of covariance) were used to assess the impact of clinical variables on muscle endpoints. Univariate and multivariate analyses were performed to determine factors that influence differences seen between groups. Age, gender, and NaMELD score were included *a priori* in the multivariate model as they were deemed likely to influence muscle endpoints. Additionally, a pre-specified statistical cut-off of *P* ≤ 0.20 was used to select additional predictors for inclusion in the multivariate model. Predictors with p<0.05 in the multivariate model were considered to be statistically significant and therefore significantly associated with the outcome. Predictors with the lowest association were sequentially removed from the model, with the likelihood ratio test performed with each iteration before arriving at the final model.

Averages were expressed as mean ± standard deviation for normally distributed variables, and as medians ± IQR for non-normally distributed variables.

This research project was approved by the local office of research and ethics, Audit/20/Austin/121.

### Results

269 patients with cirrhosis and HE were screened for this study, 127 patients were excluded due to lack of serial CT imaging within the required timeframe for analysis. 142 patients met inclusion criteria and were included in this study (see Figure 1).

### Baseline demographics

Of the 142 patients, 63 were on rifaximin with lactulose (35% female, median age 57 (51,62)), and 79 were on lactulose without rifaximin (20% female, median age 55 (51, 60)). Baseline demographics are described in Table 1.

The most common cause of liver disease in both groups was viral hepatitis, followed by alcohol, then metabolic associated fatty liver disease (MAFLD). The lactulose alone group contained a greater proportion of patients with alcohol induced cirrhosis compared to the rifaximin group (42% vs 21%: *P* = 0.009). Hepatocellular carcinoma (HCC) was present in 47% of lactulose and 47% of rifaximin patients (*P* > 0.9).

Median NaMELD scores were similar between the lactulose and rifaximin groups: 15 (12, 18) and 16 (13, 19) respectively, *P* = 0.3. The groups did not significantly differ in rates of paracentesis-dependent ascites, BMI, Charlson co-morbidity scores or presence of HCC.

There was no significant difference between lactulose versus rifaximin groups for successful elimination of hepatitis C (1.3% vs 4.8%, *P* = 0.3), or testosterone therapy (6.6% vs 6.0%, *P* >0.9) between the two CT scans.

The median time between the two CT scans was 216 (IQR 119) days in the rifaximin group and 227 (IQR 144.5) days in the lactulose group, *P* = 0.5.

### Changes in muscle mass

Using consensus gender-stratified definitions for sarcopenia for Skeletal Muscle Index (SMI) in cirrhotic cohorts (Carey et al. "A North American Expert Opinion Statement on Sarcopenia in Liver Transplantation" Hepatology (2019), 70(5): 1816-1829 and Morley et al. "Sarcopenia with limited mobility: an international consensus" J. Am. Med. Dir. Assoc. (2011), 12(6): 403-409), baseline sarcopenia was present in 59.7% of the rifaximin group and 41.8% of the lactulose group (*P* = 0.87).

Between the first and the second abdominal CT scan, the median change in SMA (cm²) in the rifaximin group was +0.74 cm² (IQR 19.7) and the median change of SMA in the lactulose group = -6.9 cm² (IQR 23.95). An increase of 0.74 cm² corresponds to a percentage increase of approximately 0.5%.

Change in SMA was analyzed by regression modelling to determine factors associated with improvement of muscle mass. Univariate analysis for SMA found that male sex (*P* <0.001), HCC presence at workup (*P* = 0.024), and greater baseline BMI (*P* = 0.001) to be associated with an increase in SMA between scans (Table 3a). Multivariate analysis including severity of liver disease that adjusted for baseline SMA was performed and found only use of rifaximin (*P* = 0.029) to be associated with improvement of SMA (Table 3b).

### Secondary outcomes

Of the lactulose group, 57 (74%) were transplanted, and 5 (6.5 %) died awaiting transplantation. In the rifaximin group, 51 (88%) were transplanted, and 2 (3.4%) died awaiting transplantation (Table 2). Median time-to-transplant was 353 days (IQR 268 days) in the rifaximin group, and 312.5 days (IQR 403.75 days) in the lactulose group. For patients who died awaiting transplant, the median time to death from listing was 265.5 days in the rifaximin group, and 282 days (IQR 252 days) in the lactulose group. There was no significant difference in rates of outcomes between the two groups (*P* = 0.07).

There was only one episode of *Clostridium difficile* infection in the rifaximin group, and none in the lactulose group.

### Conclusions

This study describes a positive association between the use of rifaximin and improvement in muscle mass over time as compared to lactulose monotherapy in a cohort of liver transplant candidates with HE. This finding was independent of baseline NaMELD score and gender, and was not influenced by potential confounders such as hepatitis C therapy or testosterone use.

Strengths of this study include the serial measurements of muscle mass in a well characterised population with excellent follow-up. Despite our cohorts being recruited from different time periods, there were no significant differences with regards to baseline variables and potential confounders, including transplant status (i.e. if patients were censored if they received a transplant between the two scans, since transplant would influence sarcopenia), use of testosterone, or hepatitis C therapy. All variables were adjusted for in the analysis by using multiple regression methods. Following this, only baseline rifaximin use (*P* = 0.029) was associated with improvement of SMA. The study used a robust, objective measure of muscle mass, namely CT SMA, which is the most commonly employed measure of muscle mass in cirrhosis and that has been well validated as a predictor of poor outcome.

In conclusion, this study demonstrates that rifaximin administration is associated with an increase in muscle mass over time in a cohort of patients with liver disease, in particular those with cirrhosis and hepatic encephalopathy. Thus, rifaximin has the potential to mitigate sarcopenia in such patients.

**Table 1: Descriptive variables**

| Characteristic | | Overall, N = 142*¹* | Lactulose alone, N = 79*¹* | Rifaximin, N = 63*¹* | p-value*²* |
|---|---|---|---|---|---|
| Follow-up skeletal muscle area (cm²) | | 160 (137, 178) | 159 (137, 174) | 162 (134, 190) | 0.3 |
| Baseline skeletal muscle area (cm²) | | 159 (139, 179) | 158 (145, 177) | 160 (131, 186) | >0.9 |
| Gender | | | | | 0.050 |
| | F | 38 (27%) | 16 (20%) | 22 (35%) | |
| | M | 104 (73%) | 63 (80%) | 41 (65%) | |
| Age (years) | | 56 (51, 61) | 55 (51, 60) | 57 (51, 62) | 0.4 |
| Viral hepatitis | | 71 (50%) | 44 (56%) | 27 (44%) | 0.2 |
| Alcohol | | 46 (33%) | 33 (42%) | 13 (21%) | 0.009 |
| Non-alcoholic fatty liver disease | | 25 (18%) | 15 (19%) | 10 (16%) | 0.7 |
| Other | | 37 (26%) | 15 (19%) | 22 (35%) | 0.027 |
| HCV eradication occurred during follow up | | | | | 0.3 |
| | N | 138 (97%) | 78 (99%) | 60 (95%) | |
| | Y | 4 (2.8%) | 1 (1.3%) | 3 (4.8%) | |
| Hepatocellular carcinoma | | | | | >0.9 |
| | N | 75 (53%) | 42 (53%) | 33 (53%) | |
| | Y | 66 (47%) | 37 (47%) | 29 (47%) | |
| MELD score | | 16(12, 19) | 15 (12, 18) | 16 (13, 19) | 0.3 |
| Ascites | | | | | >0.9 |
| | None | 44 (31%) | 24 (30%) | 20 (33%) | |
| | Diuretic-controlled | 39 (28%) | 23 (29%) | 16 (26%) | |
| | Paracentesis | 57 (41%) | 32 (41%) | 25 (41%) | |
| Charlson co-morbidity score | | 5.00 (4.00, 6.00) | 5.00 (4.00, 6.00) | 5.00 (4.00, 6.00) | 0.3 |
| Baseline height (cm) | | 173 (166, 178) | 173 (166, 179) | 172 (165, 178) | 0.5 |
| Baseline weight (kg) | | 81 (72, 92) | 84 (73, 94) | 80 (70, 90) | 0.2 |
| Baseline BMI (kg/m²) | | 27.8 (24.1, 31.1) | 28.0 (24.2, 30.9) | 26.8 (24.0, 31.2) | 0.8 |
| Testosterone therapy during follow up | | | | | >0.9 |
| | N | 118 (94%) | 71 (93%) | 47 (94%) | |
| | Y | 8 (6.3%) | 5 (6.6%) | 3 (6.0%) | |
| Duration of therapy before follow up SMA (days) | | 161 (101, 267) | 150 (87, 227) | 179 (111, 301) | 0.3 |
| Days between scans | 218 (172, 296) | 227 (173, 343) | 216 (174, 271) | 0.5 | |
| *¹* Median (IQR); n (%) | | | | | |
| *²* Wilcoxon rank sum test; Pearson's Chi-squared test; Fisher's exact test | | | | | |

**Table 2: Outcomes**

| Characteristic | | Lactulose alone, N = 79*¹* | Rifaximin, N = 63*¹* | p-value*²* |
|---|---|---|---|---|
| Transplant outcome | | | | 0.070 |
| | Delisted | 15 (19%) | 4 (6.9%) | |
| | Rejected | 0 (0%) | 1 (1.7%) | |
| | Died on list | 5 (6.5%) | 2 (3.4%) | |
| | Transplanted | 57 (74%) | 51 (88%) | |
| | Unknown | 2 | 5 | |
| *¹* n (%) | | | | |
| *²* Fisher's exact test | | | | |

**Table 3a: Regression modelling: univariate regression**

| Characteristic | | N | 95% CI | p-value |
|---|---|---|---|---|
| Gender | | 142 | | |
| | F | | - | |
| | M | | 26, 49 | <0.001 |
| Age (years) | | 142 | -0.72, 0.73 | >0.9 |
| Treatment | | 142 | | |
| | Lactulose alone | | - | |
| | Rifaximin | | -3.4, 20 | 0.2 |
| Viral hepatitis | | 141 | -5.0, 18 | 0.3 |
| Alcohol | | 141 | -3.9, 21 | 0.2 |
| Non-alcoholic fatty liver disease | | 141 | -18, 13 | 0.8 |
| Other | | 141 | -17, 9.7 | 0.6 |
| HCV eradication occurred during follow up | | 142 | | |
| | N | | - | |
| | Y | | -19, 52 | 0.4 |
| Hepatocellular carcinoma | | 141 | | |
| | N | | - | |
| | Y | | 1.8,25 | 0.024 |
| MELD score | | 141 | -1.5, 0.69 | 0.5 |
| Ascites | | 140 | | |
| | None | | - | |
| | Diuretic-controlled | | -14, 17 | 0.8 |
| | Paracentesis | | -20, 8.5 | 0.4 |
| Charlson co-morbidity score | | 141 | -2.2, 4.9 | 0.4 |
| Baseline height (cm) | | 132 | 0.98, 2.2 | <0.001 |
| Baseline weight (kg) | | 129 | 0.70, 1.3 | <0.001 |
| Baseline BMI (kg/sq m) | | 132 | 0.55, 2.1 | 0.001 |
| Baseline subjective global assessment | | 16 | | |
| | A | | - | |
| | B | | -55, 25 | 0.4 |
| | C | | -78, 39 | 0.5 |
| Testosterone therapy during follow up | | 126 | | |
| | N | | - | |
| | Y | | -9.5, 40 | 0.2 |
| Duration of rifaximin before follow up SMI (days) | | 64 | -0.05, 0.12 | 0.4 |
| Days between scans | | 142 | -0.06, 0.03 | 0.6 |

**Table 3b: Regression modelling: multivariate regression**

| Characteristic | 95% CI | p-value |
|---|---|---|
| Male gender | -4.2, 23 | 0.2 |
| Age (years) | -0.38, 0.55 | 0.7 |
| Treatment with rifaximin | 0.95, 17 | 0.029 |
| Aetiology: Alcohol | -3.7, 13 | 0.3 |
| Hepatocellular carcinoma | -8.7, 8.2 | >0.9 |
| MELD score | -1.0, 0.78 | 0.8 |
| Baseline height (cm) | -2.5, 2.2 | >0.9 |
| Baseline weight (kg) | -2.2, 2.6 | 0.9 |
| Baseline BMI (kg/sq m) | -7.0, 7.4 | >0.9 |
| Testosterone therapy during follow up | -1.6, 29 | 0.078 |

## Claims

1. Rifaximin for use in a method of treating or preventing sarcopenia in a human patient with liver disease.

2. A method of treating or preventing sarcopenia in a human patient with liver disease, the method comprising administering rifaximin to the patient.

3. Rifaximin for use according to claim 1 or the method of claim 2, wherein the human patient has cirrhosis.

4. Rifaximin for use according to any one of the preceding claims, or the method of any one of the preceding claims, wherein the patient has hepatic encephalopathy.

5. Rifaximin for use according to any one of the preceding claims, or the method of any one of the preceding claims, wherein the method comprises administering rifaximin at a dose of about 550 mg.

6. Rifaximin for use according to any one of the preceding claims, or the method of any one of the preceding claims, wherein the method comprises administering a dose of about 550 mg rifaximin twice daily.

7. Rifaximin for use according to any one of the preceding claims, or the method of any one of the preceding claims, wherein the rifaximin is administered in tablet form, preferably a film-coated tablet.

8. Rifaximin for use according to or the method of claim 7, wherein the tablet core consists of the excipients sodium starch glycolate type A, glycerol distearate, colloidal anhydrous silica, talc and microcrystalline cellulose.

9. Rifaximin for use according to or the method of claim 7 or claim 8, wherein the film coat consists of hypromellose, titanium dioxide, disodium edetate, propylene glycol and red iron oxide.

10. Rifaximin for use according to or the method of any one of the preceding claims, wherein the patient is an adult, optionally an adult aged 50 years or older.

11. Rifaximin for use according to or the method of any one of the preceding claims, wherein the method results in at least maintenance of skeletal muscle area.

12. Rifaximin for use according to or the method of any one of the preceding claims, wherein the method results in an increase in skeletal muscle area of at least about 0.25 cm², at least about 0.50 cm², or at least about 0.70 cm².

13. Rifaximin for use according to or the method of any one of the preceding claims, wherein the method results in an increase in skeletal muscle area of at least 0.25%, at least 0.50%, at least 0.75%, at least 1.0% or at least 2.0%, preferably at least 0.5%.

14. Rifaximin for use according to or the method of any one of the preceding claims, wherein the method further comprises administering lactulose to the patient.

15. Use of rifaximin in the manufacture of a medicament for treating or preventing sarcopenia in a human patient with liver disease.
